# FASCICULE DE BREVET EUROPEEN

(11) **EP 0 719 776 B1**
(45) Date de publication et mention de la délivrance du brevet: **04.11.1998**
(21) Numéro de dépôt: 95402841.1
(22) Date de dépôt: 18.12.1995
(51) Int. Cl.: C07D 455/02

(54) **Dérivés d'acide 8-oxo-5,8-dihydro-6H-dibenzo[a,g]quinolizine-13-propanoique comme ligands des récepteurs benzodiazépiniques associés aux récepteurs GABAA**
8-Oxo-5,8-dihydro-6H-dibenzo[a,g]chinolizin-13-propansäure-Derivate als Liganden der mit GABAA Rezeptoren verbundenen Benzodiazepine Rezeptoren
8-Oxo-5,8-dihydro-6H-dibenzo[a,g]quinolizine-13-propanoic acid derivatives as ligands of benzodiazepine receptors associated with GABAA receptors

(30) Priorité: 29.12.1994 FR 9415836
(43) Date de publication de la demande: 03.07.1996
(73) Titulaire: SYNTHELABO, 92350 Le Plessis Robinson (FR)
(72) Inventeur: Marabout, Benoît, F-91380 Chilly Mazarin (FR); Sevrin, Mireille, F-75014 Paris (FR); Froissant, Jacques, F-41160 Moree (FR); Dachary, Emmanuelle, F-94400 Vitry sur Seine (FR)
(74) Mandataire: Ludwig, Jacques

(56) Documents cités:
- Aucun document pertinent relevé

## Description

La présente invention a pour objet des dérivés d'acide 8-oxo-5,8-dihydro-6*H*-dibenzo[*a,g*]quinolizine-13-propanoïque, leur préparation et leur application en thérapeutique.

Les composés de l'invention répondent à la formule générale (I) 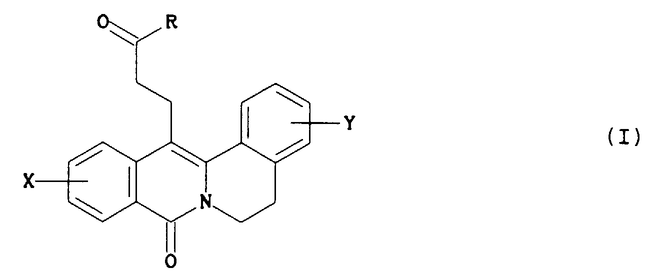 dans laquelle
X représente un atome d'hydrogène ou d'halogène ou un groupe alkyle en C₁-C₃, un ou deux groupes alcoxy en C₁-C₃ ou un groupe trifluorométhyle,
Y représente un atome d'hydrogène ou d'halogène, un groupe alkyle en C₁-C₃ ou un groupe alcoxy en C₁-C₃,
R représente un groupe hydroxy, un groupe méthoxy ou un groupe de formule générale NR₂R₃ dans laquelle R₂ et R₃, indépendamment l'un de l'autre, représentent chacun un atome d'hydrogène, un groupe alkyle en C₁-C₄, un groupe 2-méthoxyéthyle, un groupe 2-aminoéthyle, un groupe 3-aminopropyle, un groupe 2-(diméthylamino)éthyle, un groupe 3-(diméthylamino)propyle ou un groupe 2-pipéridin-1-yléthyle, ou bien encore R₂ et R₃ forment ensemble, et avec l'atome d'azote qui les porte, un cycle morpholinyle, pyrrolidinyle ou pipérazinyle éventuellement substitué en position 4 par un groupe méthyle ou (1,1-diméthyléthoxy)carbonyle.

Les composés peuvent éventuellement exister à l'état de bases ou de sels d'addition à des acides.

Conformément à l'invention, on peut préparer les composés de formule générale (I) par un procédé illustré par le schéma 1 qui suit.

On fait réagir un anhydride de formule générale (II), dans laquelle X est tel que défini ci-dessus, avec une imine de 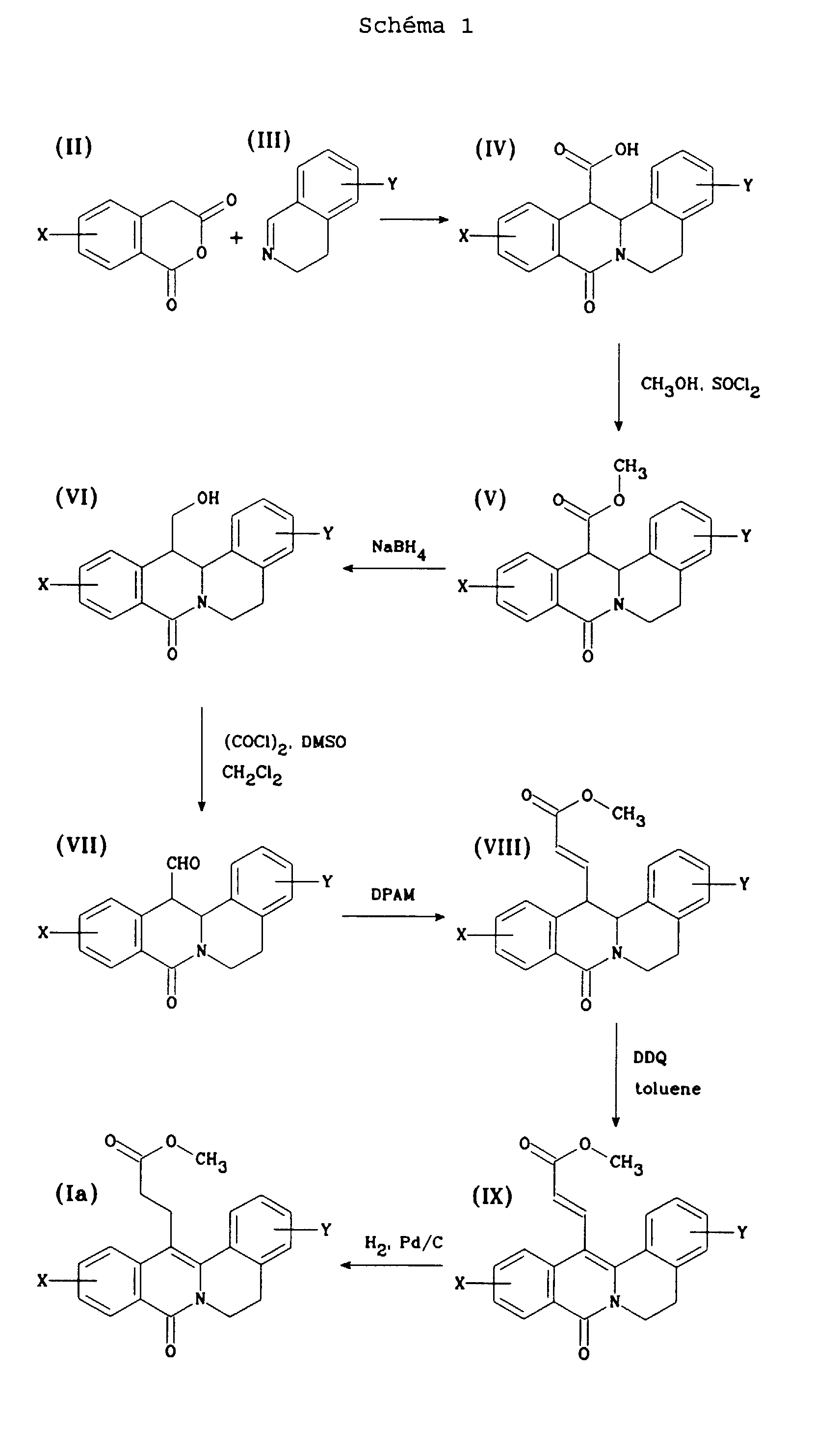 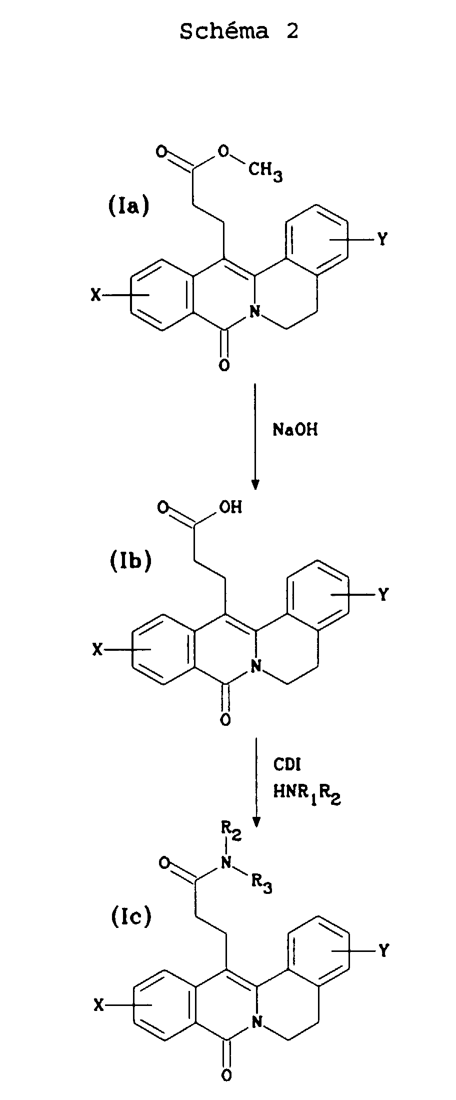 formule générale (III), dans laquelle Y est tel que défini ci-dessus, dans un solvant aromatique, par exemple le toluène, à une température de 70 à 100°C. On obtient un acide de formule générale (IV) que l'on estérifie par le chlorure de thionyle dans le méthanol, à une température de 20 à 50°C, puis on transforme l'ester ainsi obtenu, de formule générale (V), en alcool de formule générale (VI), par exemple par un hydrure mixte tel que le borohydrure de sodium, dans un solvant éthéré tel que le tétrahydrofurane. Ensuite on oxyde l'alcool en aldéhyde de formule générale (VII), par exemple selon la méthode de Swern, on traite l'aldéhyde par le (diméthoxyphosphinyl)acétate de méthyle (DPAM), dans un solvant éthéré tel que le tétrahydrofurane, à une température de 20 à 75°C, on isole l'ester de formule générale (VIII) ainsi obtenu et on l'oxyde en ester de formule générale (IX) au moyen d'une quinone, par exemple la 2,3-dichloro-5,6-dicyanocyclohexa-2,5-diène-1,4-dione, par exemple dans un solvant aromatique tel que le toluène, à une température de 70 à 110°C, et finalement on soumet l'ester de formule générale (IX) à une hydrogénation en présence de charbon palladié, pour obtenir l'ester de formule générale (Ia), qui correspond à la formule générale (I) où R représente un groupe méthoxy.

Cet ester peut ensuite être transformé comme illustré par le schéma 2 : si l'on désire un composé de formule générale (I) où R représente un groupe hydroxy, on soumet l'ester de formule générale (Ia) à une hydrolyse en milieu basique pour obtenir un acide de formule générale (Ib) et, si l'on désire un composé de formule générale (I) où R représente un groupe de formule générale NR₂R₃ on traite l'acide de formule générale (Ib) avec une amine de formule générale HNR₂R₃ en passant par l'imidazolide intermédiaire préparé *in situ* au moyen de *N,N'*-carbonyldiimidazole.

Dans le cas des composés de formule générale (I) où X et Y sont différents d'un groupe alcoxy, on peut mettre en oeuvre un autre procédé illustré par le schéma 3.

On soumet la dibenzo[*a,g*]quinolizine de formule générale (X) à un traitement par l'oxychlorure de phosphore, dans le *N,N*-diméthylformamide, à une température de 20 à 130°C, pour obtenir l'aldéhyde de formule générale (XI), puis on traite ce dernier avec le (diméthoxyphosphinyl)acétate de méthyle, dans un solvant éthéré tel que le tétrahydrofurane, à une température de 20 à 65°C pour obtenir l'ester de formule générale (IX), que l'on finit de traiter comme décrit à propos du schéma 1.

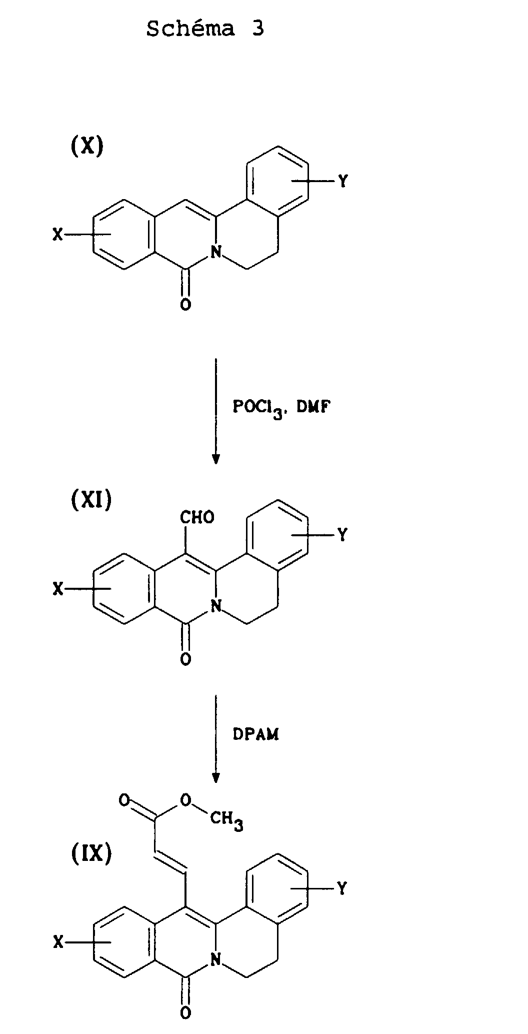

L'anhydride de départ, de formule générale (II), est disponible dans le commerce lorsque X représente l'hydrogène et, dans les autres cas, il peut être préparé selon une méthode telle que celle décrite dans *Arch. Pharm.* (1991) **324** 509-518.
Les imines substituées de formule générale (III) peuvent être préparées par des méthodes analogues à celles décrites dans *Synthesis* (1974) **4** 288-289 et *Heterocycles* (1982) **19**(4) 653-656.
Les quinolizines de formule générale (X) peuvent être préparées par des méthodes analogues à celles décrites dans *Synthesis* (1980) **10** 845-847 et dans *Tetrahedron Lett.* (1992) **33**(38) 5653-5654.

Les exemples qui vont suivre illustrent la préparation de quelques composés de l'invention. Les microanalyses élémentaires, et les spectres I.R. et R.M.N. confirment les structures des composés obtenus. Les numéros indiqués entre parenthèses dans les titres des exemples correspondent à ceux de la 1ère colonne du tableau 1 donné plus loin. Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

### Exemple 1 (Composé N°1)

### 8-Oxo-5,8-dihydro-6H-dibenzo[a, g]quinolizine-13-propanoate de méthyle.

1.1. 8-Oxo-5,8-dihydro-6*H*-dibenzo[*a, g*]quinolizine-13-carboxaldéhyde.
   On refroidit à 0°C, sous atmosphère d'argon, 150 ml de *N,N*-diméthylformamide sec, on ajoute, goutte à goutte, 5 ml (53,6 mmoles) d'oxychlorure de phosphore, on agite le mélange pendant 30 min à température ambiante, on ajoute 5 g (20,2 mmoles) de 5,6-dihydro-8*H*-dibenzo[*a,g*]quinolizin-8-one, on chauffe le mélange progressivement jusqu'à 110°C et on le maintient à cette température pendant 6h.
   On le refroidit à température ambiante, on évapore le solvant sous pression réduite, on ajoute au résidu de la glace et de la soude à 30%, on l'extrait au dichlorométhane, on sèche la phase organique sur sulfate de sodium, on la filtre et on évapore le solvant sous pression réduite. On purifie le résidu par chromatographie sur colonne de gel de silice en éluant avec un mélange de cyclohexane/dichlorométhaneallant de 100/0 à 0/100, puis avec un mélange de dichlorométhane/acétate d'éthyle allant de 100/0 à 80/20.
   Après recristallisation dans le cyclohexane on isole 3,6 g (13,08 mmoles) d'aldéhyde sous forme de solide blanc.
   Point de fusion : 209-210°C.
1.2. (*E*)-3-(8-Oxo-5,8-dihydro-6*H*-dibenzo[*a,g*]quinolizin-13-yl)prop-2-énoate de méthyle.
   Sous atmosphère d'argon on lave 0,5 g (12,5 mmoles) d'une suspension à 60% d'hydrure de sodium avec du pentane, puis on en prépare une suspension dans 150 ml de tétrahydrofurane sec, on refroidit le mélange avec un bain de glace, on ajoute, goutte à goutte, 2 g (11 mmoles) de (diméthoxyphosphinyl)acétate de méthyle, on agite le mélange pendant 15 min à 0°C, on ajoute 2,78 g (10,1 mmoles) de 8-oxo-5,8-dihydro-6*H*-dibenzo[*a,g*]quinolizine-13-carboxaldéhyde, on chauffe le mélange progressivement jusqu'au reflux, et on maintient le reflux pendant 6h.
   On refroidit le mélange à température ambiante, on ajoute quelques ml de méthanol, on évapore les solvants sous pression réduite, on ajoute au résidu de la glace, 200 ml de dichlorométhane et de l'acide chlorhydrique 1M, on sépare la phase organique, on la sèche sur sulfate de sodium, on la filtre, et on évapore le solvant sous pression réduite. On purifie le résidu par chromatographie sur colonne de gel de silice en éluant avec un mélange de dichlorométhane/acétate d'éthyle allant de 100/0 à 80/20 et on recristallise le produit dans le cyclohexane. On isole 2,78 g (8,4 mmoles) d'ester α,β-insaturé sous forme de cristaux blancs.
   Point de fusion : 201-202,5°C.
1.3. 8-Oxo-5,8-dihydro-6*H*-dibenzo[*a,g*]quinolizine-13-propanoate de méthyle.
   A une solution de 2,8 g (8,4 mmoles) de (*E*)-3-(8-oxo-5,8-dihydro-6*H*-dibenzo[*a,g*]quinolizin-13-yl)prop-2-énoate de méthyle dans 150 ml d'acide acétique, on ajoute 0,23 g de charbon palladié à 5%, et on soumet la suspension à une hydrogénation dans un appareil de Parr sous une pression d'environ 0,32 MPa à température ambiante pendant 30 min puis entre 40 et 45°C pendant 2h30.
   On sépare le catalyseur par filtration, on concentre le filtrat sous pression réduite, on ajoute au résidu 250 ml de dichlorométhane, de l'eau glacée et de la solution saturée d'hydrogénocarbonate de sodium, on sépare la phase organique, on la lave à l'eau, on la sèche sur sulfate de sodium, on la filtre et on évapore le solvant sous pression réduite. On purifie le résidu par chromatographie sur colonne de gel de silice en éluant avec un mélange de dichlorométhane/acétate d'éthyle de 100/0 à 70/30, puis avec un mélange de dichlorométhane/méthanol de 95/5 à 90/10, et on obtient 2,8 g (8,4 mmoles) de produit huileux. Après recristallisation dans le cyclohexane on isole 2,5 g (7,5 mmoles) d'ester sous forme de cristaux blancs.
   Point de fusion : 157-158,5°C.

### Exemple 2 (Composé N°2)

### Acide 8-oxo-5,8-dihydro-6H-dibenzo[a,g]quinolizine-13-propanoïque.

Dans un ballon de 100 ml on introduit 2,45 g (7,35 mmoles) de 8-oxo-5,8-dihydro-6*H*-dibenzo[*a, g*]quinolizine-13-propanoate de méthyle en solution dans 40 ml d'éthanol, on ajoute 2 ml de soude aqueuse à 30% et on chauffe le mélange au reflux pendant 3h.
On évapore le solvant sous pression réduite, on reprend le résidu avec de l'eau et de l'acide chlorhydrique à 30%, on collecte le solide qui précipite par filtration, on le lave à l'eau et on le sèche.
On obtient 2,2 g (6,89 mmoles) d'acide.
Point de fusion : 269-271 (décomposition).

### Exemple 3 (Composé N°12)

### N,N-Diméthyl-8-oxo-5,8-dihydro-6H-dibenzo[a,g]quinolizine-13-propanamide.

Dans un ballon de 250 ml, placé sous atmosphère d'argon, on prépare une suspension de 1,2 g (3,76 mmoles) d'acide 8-oxo-5,8-dihydro-6*H*-dibenzo[*a,g*] quinolizine-13-propanoïque dans 100 ml de dichlorométhane, on ajoute 1,2 g (7,4 mmoles) de *N,N'*-carbonyldiimidazole et on agite le mélange à température ambiante pendant 2h.
On sature le milieu avec de la diméthylamine gazeuse pendant 1 min et on laisse le mélange sous agitation pendant 12h. On évapore le solvant sous pression réduite, on ajoute au résidu 200 ml de dichlorométhane, on lave la solution avec de l'acide chlorhydrique 1N, puis avec de l'eau, puis avec de la soude 1N, puis encore avec de l'eau, on la sèche sur sulfate de sodium, on la filtre, on évapore le solvant sous pression réduite, et on purifie le résidu par chromatographie sur colonne de gel de silice en éluant avec un mélange de dichlorométhane/méthanol de 100/0 à 90/10.
Après recristallisation dans un mélange d'acétonitrile/di-chlorométhane on obtient 0,93 g (2,68 mmoles) de solide blanc.
Point de fusion : 192,5-193,5°C.

### Exemple 4 (Composé N°38)

### Acide 10-méthoxy-8-oxo-5,8-dihydro-6H-dibenzo[a,g]quinolizine-13-propanoïque.

4.1. Acide 10-méthoxy-8-oxo-5,8,13,13a-tétrahydro-6*H*-dibenzo [*a, g*]quinolizine-13-carboxylique.
   Dans un ballon de 500 ml on introduit 8,1 g (42,15 mmoles) d'anhydride 7-méthoxyhomophtalique en solution dans 350 ml de toluène, on ajoute 5,8 g (44,2 mmoles) de 3,4-dihydroisoquinoléine et on chauffe le mélange au reflux pendant 3h. On le laisse revenir à température ambiante et on le laisse au repos pendant une nuit.
   On collecte les cristaux qui se sont formés, on les lave à l'éther diéthylique et on les sèche sous vide.
   On obtient 12,75 g (39,43 mmoles) d'acide en mélange *trans/cis* à environ 90/10.
   Point de fusion : 227-232°C.
4.2. *Trans*-10-méthoxy-8-oxo-5,8,13,13a-tétrahydro-6*H*-dibenzo[*a, g*] quinolizine-13-carboxylate de méthyle.
   Dans un ballon tricol de 500 ml on prépare une suspension de 14 g (43,3 mmoles) d'acide 10-méthoxy-8-oxo-5,8,13,13a-tétrahydro-6*H*-dibenzo [*a,g*] quinolizine-13-carboxylique dans 100 ml de méthanol, on ajoute, goutte à goutte, 3,2 ml (44,11 mmoles) de chlorure de thionyle et on chauffe le mélange au reflux pendant 4h.
   On le laisse au repos à température ambiante une nuit.
   On collecte les cristaux qui se sont formés, on les lave à l'éther diéthylique et on les sèche sous vide.
   On obtient 11,5 g (34,08 mmoles) d'ester *trans* pur.
   Point de fusion : 154,5-157,5°C.
4.3. Trans-13-(hydroxyméthyl)-10-méthoxy-5,6,13,13a-tétrahydro-8*H*-dibenzo[*a,g*]quinolizin-8-one.
   Dans un ballon de 250 ml on prépare une suspension à partir de 9,85 g (29,2 mmoles) de trans-10-méthoxy-8-oxo-5,8,13,13a-tétrahydro-6*H*-dibenzo [*a, g*]quinolizine-13-carboxylate de méthyle et de 75 ml de tétrahydrofurane, on ajoute successivement 4,68 g (146 mmoles) de méthanol et 5,52 g (146 mmoles) de borohydrure de sodium et on agite le mélange à température ambiante pendant 4h.
   On l'hydrolyse en ajoutant 100 ml d'eau, on l'extrait avec trois fois 150 ml de dichlorométhane, on sèche la phase organique sur sulfate de sodium, on évapore le solvant sous pression réduite et on purifie le résidu par chromatographie sur colonne de gel de silice en éluant avec un mélange de dichlorométhane/acétate /acétate d'éthyle de 100/0 à 50/50.
   Après évaporation des solvants on reprend les cristaux avec de l'éther diéthylique et on obtient finalement 8,3 g (26,83 mmoles) d'alcool *trans*.
   Point de fusion : 180-181,5°C.
4.4. 10-Méthoxy-8-oxo-5,8,13,13a-tétrahydro-6*H*-dibenzo[a, g] quinolizine-13-carboxaldéhyde.
   Dans un ballon tricol de 500 ml, placé sous atmosphère d'argon, on introduit 20,2 g (159 mmoles) de chlorure d'oxalyle et 100 ml de dichlorométhane, on refroidit l'ensemble à -70°C, on ajoute, goutte à goutte, 16,36 g (209 mmoles) de diméthylsulfoxyde en solution dans 50 ml de dichlorométhane, et on agite le tout à -70°C pendant 15 min.
   On ajoute, en l'espace de 30 min, 8,2 g (26,68 mmoles) de *trans*-13-(hydroxyméthyl)-10-méthoxy-5,6,13,13a-tétrahydro-8*H*-dibenzo[*a,g*]quinolizin-8-one en solution dans 150 ml de dichlorométhane et on agite le mélange à -60°C pendant 30 min.
   On ajoute 21,2 g (209 mmoles) de triéthylamine en solution dans 50 ml de dichlorométhane et on laisse le mélange revenir à température ambiante.
   On le verse sur 500 ml de solution aqueuse saturée de chlorure de sodium et on extrait avec trois fois 200 ml de dichlorométhane. On sèche la phase organique sur sulfate de sodium, on la filtre et on évapore les solvants sous pression réduite. On purifie le résidu par chromatographie sur colonne de gel de silice en éluant avec un mélange de dichlorométhane/cyclohexane 2/1 puis 1/0, puis avec un mélange de dichlorométhane/acétate d'éthyle de 90/10 à 50/50.
   Après recristallisation dans un mélange d'acétate d'éthyle et d'éther de pétrole on obtient 8,02 g (26,09 mmoles) d'aldéhyde en mélange *trans/cis* à 80/20.
   Point de fusion : 146-149°C.
4.5. (*E*)-3-(10-méthoxy-8-oxo-5,8,13,13a-tétrahydro-6*H*-dibenzo[*a,g*]quinolizin-13-yl)prop-2-énoate de méthyle.
   Dans un ballon tricol de 500 ml on lave au pentane 1,3 g (32,5 mmoles) d'hydrure de sodium en suspension à 60% dans l'huile, on les met en suspension dans 200 ml de tétrahydrofurane, on refroidit la suspension à 0°C, on ajoute, goutte à goutte, 5,2 g (28,55 mmoles) de (diméthoxyphosphinyl)acétate de méthyle et on poursuit l'agitation à environ 10°C pendant 20 min.
   On ajoute 8 g (26 mmoles) de 10-méthoxy-8-oxo-5,8,13,13a-tétrahydro-6*H*-dibenzo [*a,g*] quinolizine-13-carboxaldéhyde et on chauffe le mélange au reflux pendant 4h30.
   On le laisse revenir à température ambiante, on ajoute quelques gouttes de méthanol, on évapore les solvants sous pression réduite, on ajoute au résidu 250 ml d'eau et on extrait avec trois fois 150 ml de dichlorométhane. On sèche la phase organique sur sulfate de sodium, on la filtre, on évapore le solvant sous pression réduite et on purifie le résidu par chromatographie sur colonne de gel de silice en éluant avec un mélange d'acétate d'éthyle/cyclohexane 1/2 à 3/1, et on obtient 6,55 g (18,02 mmoles) d'ester sous forme d'huile qu'on utilise tel quel dans l'étape suivante.
4.6. (*E*)-3-(10-méthoxy-8-oxo-5,8-dihydro-6*H*-dibenzo[*a,g*]-quinolizin-13-yl)prop-2-énoate de méthyle.
   Dans un ballon de 500 ml on dissout 5,5 g (15,13 mmoles) de (*E*)-3-(10-méthoxy-8-oxo-5,8,13,13a-tétrahydro-6*H*-dibenzo[*a,g*]quinolizin-13-yl)prop-2-énoate de méthyle dans 150 ml de toluène, on ajoute 5,15 g (22,7 mmoles) de 2,3-dichloro-5,6-dicyanocyclohexa-2,5-diène-1,4-dione et on chauffe le mélange au reflux pendant 2h30.
   On le laisse refroidir à température ambiante, on évapore le solvant sous pression réduite et on purifie le résidu par chromatographie sur colonne de gel de silice en éluant avec un mélange de dichlorométhane/acétate d'éthyle de 100/0 à 80/20. On reprend l'huile obtenue dans un mélange d'éther diéthylique et d'éther de pétrole, et on isole 3,31 g (9,2 mmoles) de composé sous forme de cristaux jaune-orange.
   Point de fusion : 149-152°C.
4.7. 10-Méthoxy-8-oxo-5,8-dihydro-6*H*-dibenzo[*a,g*]quinolizine-13-propanoate de méthyle.
   A une solution de 3,5 g (9,68 mmoles) de (*E*)-3-(10-méthoxy-8-oxo-5,8-dihydro-6*H*-dibenzo[*a, g*]quinolizin-13-yl)prop-2-énoate de méthyle dans 100 ml d'acide acétique on ajoute 0,5 g de charbon palladié à 5% et on soumet la suspension à une hydrogénation dans un appareil de Parr sous une pression d'environ 0,32 MPa entre 50 et 60°C pendant 3h.
   On laisse refroidir à température ambiante, on sépare le catalyseur par filtration, on concentre le filtrat sous pression réduite, on ajoute 250 ml de dichlorométhane au résidu, on lave la solution avec une solution aqueuse saturée d'hydrogénocarbonate de sodium, on la sèche sur sulfate de sodium, on la filtre et on évapore les solvants sous pression réduite. On purifie le résidu par chromatographie sur colonne de gel de silice en éluant avec un mélange de dichlorométhane/acétate d'éthyle de 100/0 à 50/50 et on obtient 3,4 g (9,36 mmoles) d'ester sous forme d'huile qu'on utilise tel quel dans l'étape suivante.
4.8. Acide 10-méthoxy-8-oxo-5,8-dihydro-6*H*-dibenzo[*a,g*]quinolizine-13-propanoïque.
   Dans un ballon de 100 ml on introduit 3,4 g (9,36 mmoles) de 10-méthoxy-8-oxo-5,8-dihydro-6*H*-dibenzo[*a,g*]quino1izine-13-propanoate de méthyle en solution dans 50 ml d'éthanol, on ajoute 3 ml de soude aqueuse à 30%, et on chauffe le mélange au reflux pendant 3h.
   On évapore les solvants sous pression réduite, on ajoute au résidu de l'eau et de l'acide chlorhydrique à 30%, on collecte le solide qui précipite, on le lave à l'eau et on le sèche.
   On obtient finalement 3,0 g (8,58 mmoles) d'acide.
   Point de fusion : 260-264°C.

### Exemple 3 (Composé N°40)

### 10-Méthoxy-N-méthyl-8-oxo-5,8-dihydro-6H-dibenzo[a,g]quinolizine-13-propanamide.

Dans un ballon de 250 ml, sous atmosphère d'argon, on prépare une suspension de 1 g (2,86 mmoles) d'acide 10-méthoxy-8-oxo-5,8-dihydro-6*H*-dibenzo[*a,g*]quinolizine-13-propanoique dans 100 ml de dichlorométhane, on ajoute 0,93 g (5,72 mmoles) de *N,N'*-carbonyldiimidazole et on agite le mélange à température ambiante pendant 2h.
Ensuite on sature le mélange avec de la méthylamine gazeuse pendant 1 min et on poursuit l'agitation pendant 22h.
On évapore le solvant sous pression réduite, on ajoute au résidu 200 ml de dichlorométhane, on lave la solution avec de l'eau, puis avec de l'acide chlorhydrique 1N, puis avec de la soude 1N, puis encore avec de l'eau, on la sèche sur sulfate de sodium, on la filtre, on évapore le solvant sous pression réduite et on purifie le résidu par chromatographie sur colonne de gel de silice en éluant avec un mélange dichlorométhane/méthanol de 100/0 à 97/3.
Après recristallisation dans l'acétate d'éthyle on obtient finalement 0,43 g (1,19 mmole) de solide blanc.
Point de fusion : 182-183°C.

Le tableau qui suit illustre les structures chimiques et les propriétés physiques de quelques composés selon l'invention.

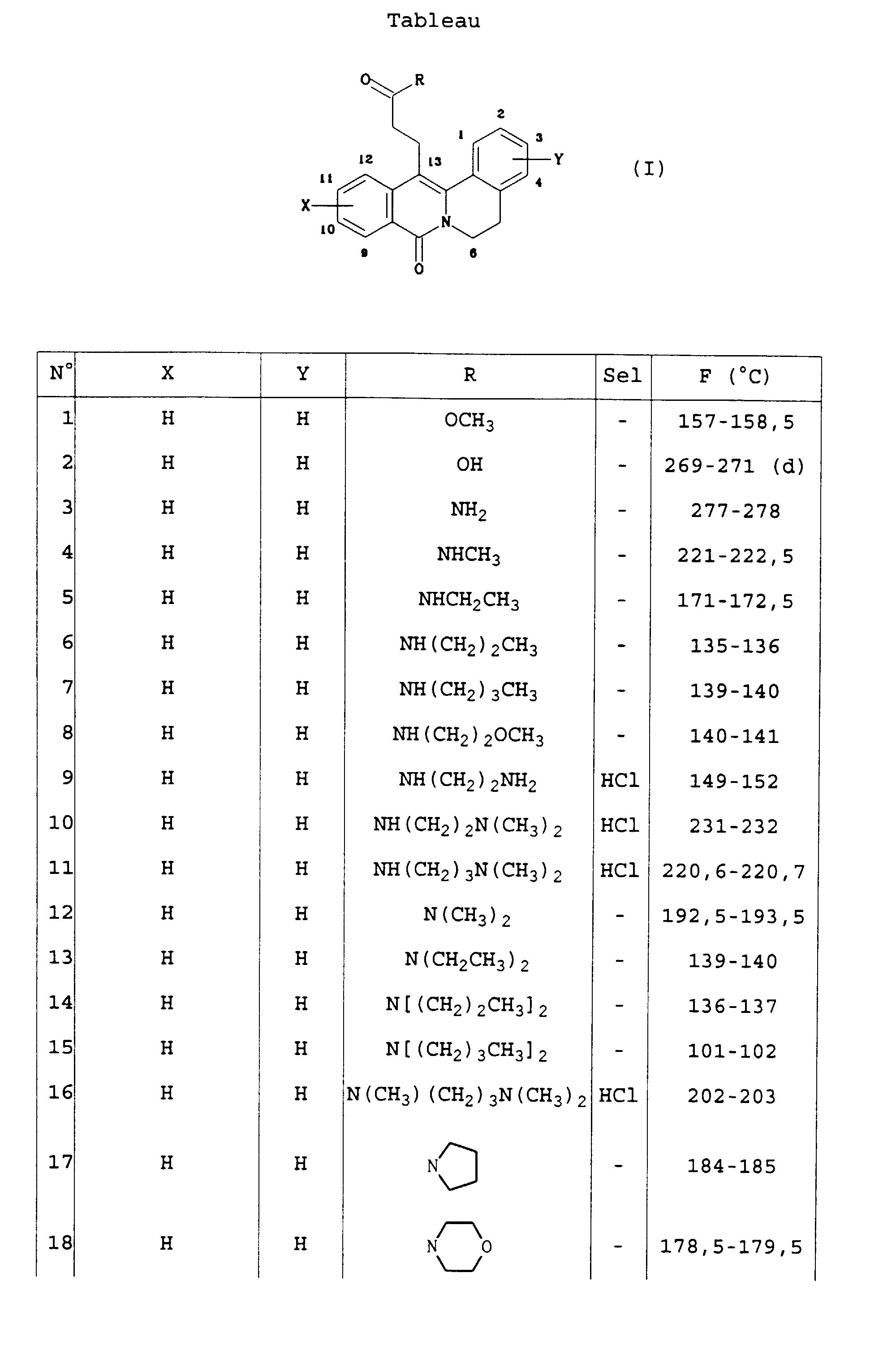

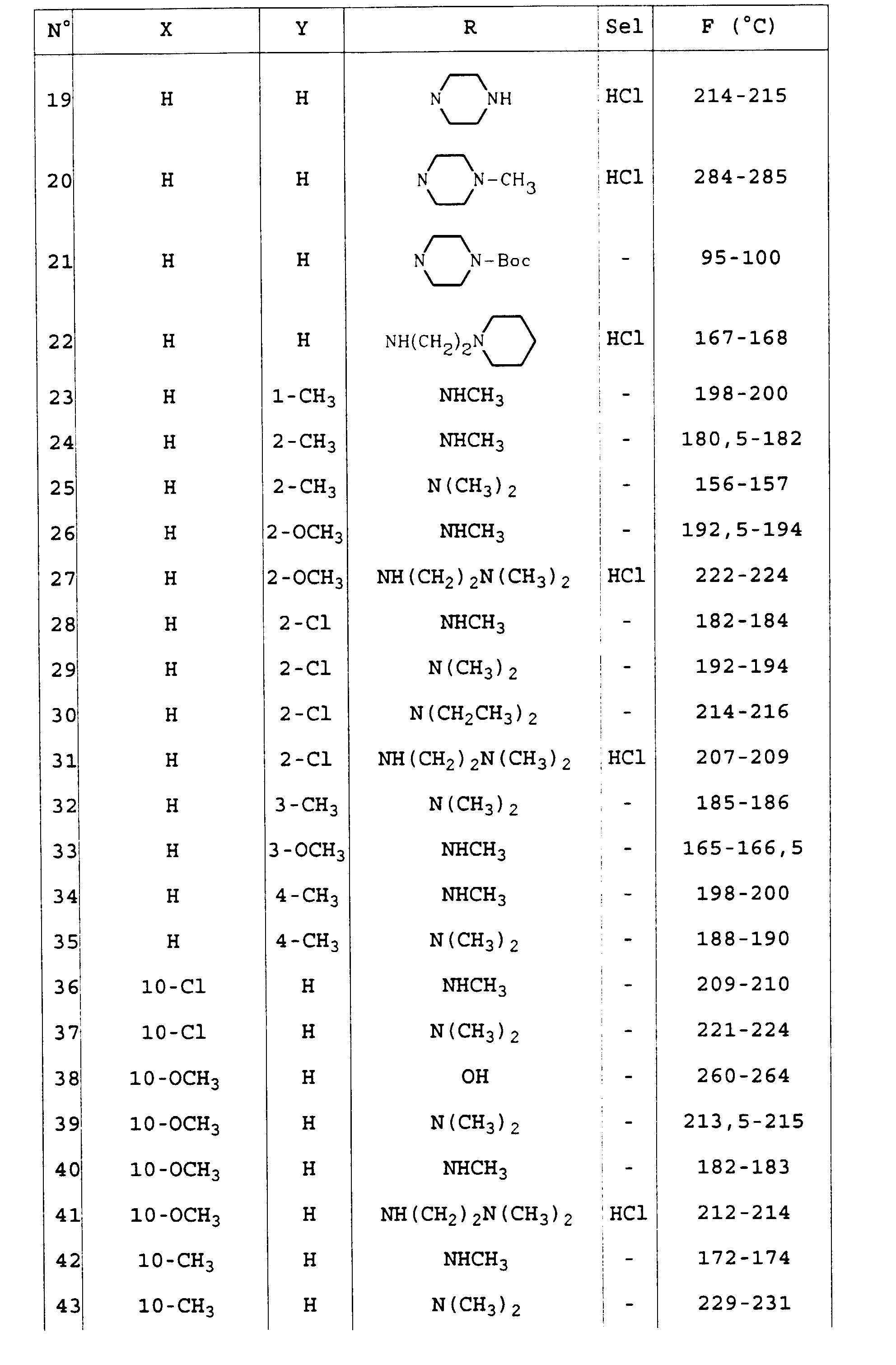

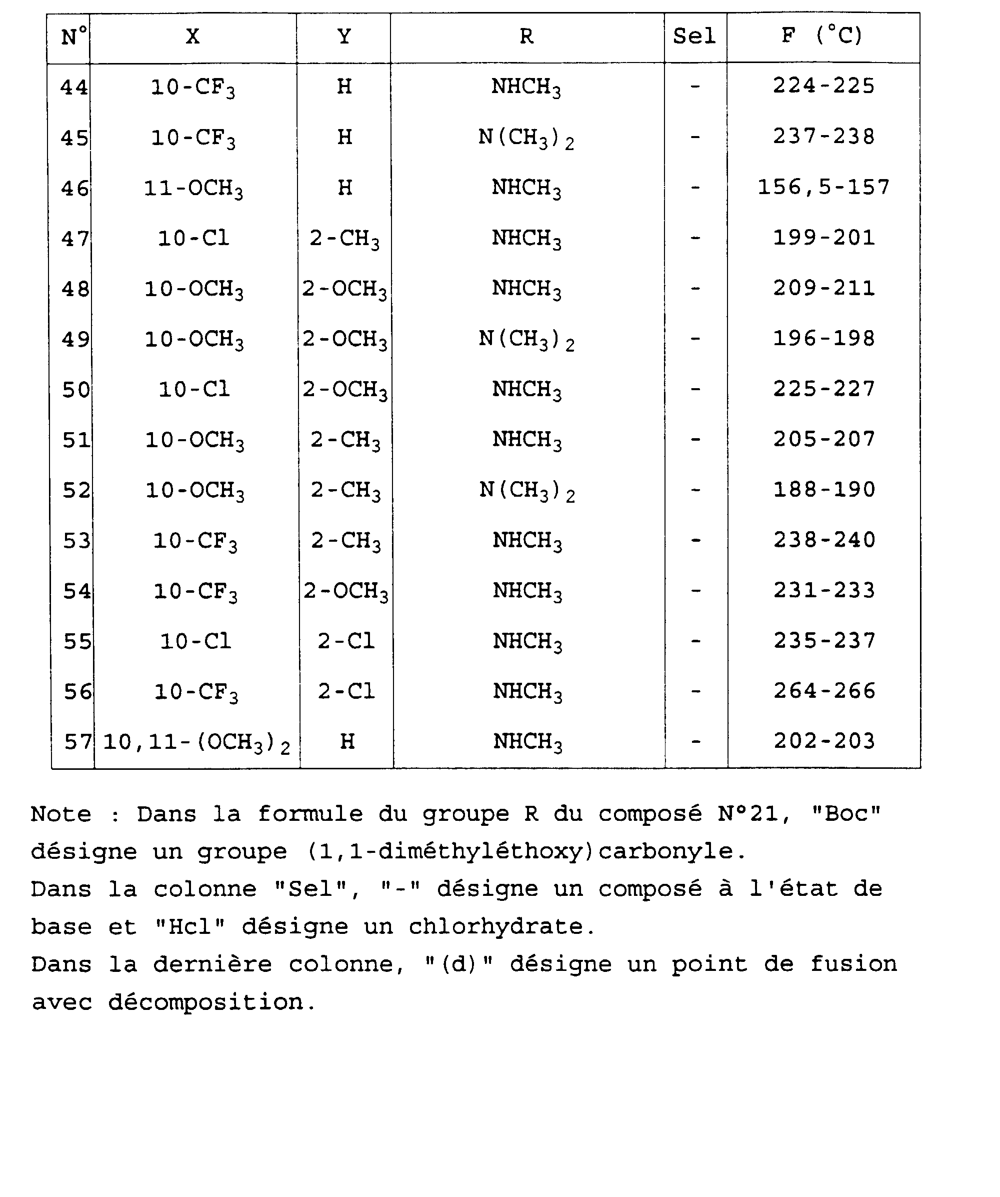

Les composés de l'invention ont été soumis à des essais pharmacologiques qui ont mis en évidence leur intérêt comme substances à activités thérapeutiques.

### Etude de la liaison membranaire vis-à-vis d'une population de récepteurs ω (benzodiazépiniques) associée aux récepteurs GABA_{A} contenant la sous-unité α₅.

Ces récepteurs peuvent être marqués sélectivement dans des membranes d'hippocampe de rat incubées en présence de [³H]flumazénil et de zolpidem 5 µM (afin de masquer les autres sous-types de récepteurs ω).

Les composés ont fait l'objet d'une étude *in vitro* quant à leur affinité pour ces récepteurs marqués par le [³H]flumazénil.

Les animaux utilisés sont des rats mâles OFA (Iffa Credo) de 200 à 250 g. Après décapitation on prélève l'hippocampe et on le broie au moyen d'un appareil Ultra-Turrax™ ou Polytron™ pendant 20 s à 6/10 de la vitesse maximale dans 80 volumes de tampon Tris 50 mM à un pH ajusté à 7,4 avec de l'acide chlorhydrique, et contenant 120 mM de chlorure de sodium et 5 mM de chlorure de potassium (5 mM).

La liaison avec le [³H]flumazénil (1 nM ; activité spécifique : 80-87 Ci/mmole ; Du Pont de Nemours / New England Nuclear) est déterminée par incubation de 200 µl de suspension de membranes dans un volume final de 1 ml de tampon contenant 5 µM de zolpidem et le composé à tester. Après une incubation de 45 min à 0°C on récupère les membranes par filtration sur filtres Whatman GF/B™ qu'on lave deux fois avec 5 ml de tampon glacé. On mesure la quantité de radioactivité retenue par le filtre par scintigraphie liquide.

La liaison spécifique du [³H]flumazénil est définie comme la quantité de radioactivité retenue sur les filtres et pouvant être inhibée par co-incubation avec le flunitrazepam 1 µM.

Pour chaque concentration de composé étudiée on détermine le pourcentage d'inhibition de la liaison du [³H]flumazénil, puis la concentration CI₅₀, concentration qui inhibe 50% de la liaison spécifique.

Les composés de l'invention les plus actifs dans cet essai ont une CI₅₀ de l'ordre de 1 à 100 nM.

### Etude des liaisons membranaires vis-à-vis des récepteurs ω₂ (benzodiazépiniques de type II) associés aux récepteurs GABA_{A} contenant majoritairement les sous-unités α₂ et α₃.

L'affinité des composés pour les récepteurs ω₂ de la moelle épinière a été déterminée selon une variante de la méthode décrite par S. Z. Langer et S. Arbilla dans *Fund. Clin. Pharmacol.* (1988) **2** 159-170, avec utilisation de [³H]flumazénil au lieu de [³H]diazepam comme radioligand.

On homogénéise le tissu de la moelle épinière pendant 60 s dans 30 volumes de tampon glacé (50 ml Tris/HCl, pH 7,4, NaCl 120 mM, KCl 5 mM) puis, après dilution à 1/3, on fait incuber la suspension avec du [³H]flumazénil (activité spécifique : 78 Ci/mmole ; New England Nuclear) à une concentration de 1 nM et avec les composés de l'invention, à différentes concentrations, dans un volume final de 525 µl. Après 30 min d'incubation à 0°C on filtre les échantillons sous vide sur des filtres Whatman GF/B™ et on les lave immédiatement avec du tampon glacé. La liaison spécifique du [³H]flumazénil est déterminée en présence de diazepam 1 µM non marqué. On analyse les données selon les méthodes usuelles et on calcule la CI₅₀, concentration qui inhibe 50% de la liaison du [³H]flumazénil.

Les CI₅₀ des composés de l'invention se situent, dans cet essai, entre 1 et 500 nM.

### Etude des liaisons membranaires vis-à-vis des récepteurs ω₁ (benzodiazépiniques de type I) associés aux récepteurs GABA_{A} contenant la sous-unité α ₁.

L'affinité des composés pour les récepteurs ω₁ du cervelet a été déterminée selon une variante de la méthode décrite par S . Z. Langer et S. Arbilla dans *Fund. Clin. Pharmacol.* (1988) **2** 159-170, avec utilisation de [³H]flumazénil au lieu de [³H]diazepam comme radioligand.

On homogénéise le tissu du cervelet pendant 60 s dans 120 volumes de tampon glacé (50 ml Tris/HCl, pH 7,4, NaCl 120 mM, KCl 5 mM) puis, après dilution à 1/3, on fait incuber la suspension avec du [³H]flumazénil (activité spécifique : 78 Ci/mmole ; New England Nuclear) à une concentration de 1 nM et avec les composés de l'invention, à différentes concentrations, dans un volume final de 525 µl. Après 30 min d'incubation à 0°C on filtre les échantillons sous vide sur des filtres Whatman GF/B™ et on les lave immédiatement avec du tampon glacé. La liaison spécifique du [³H]flumazénil est déterminée en présence de diazepam 1 µM non marqué. On analyse les données selon les méthodes usuelles et on calcule la CI₅₀, concentration qui inhibe 50% de la liaison du [³H]flumazénil.

Les CI₅₀ des composés de l'invention se situent, dans cet essai, entre 1 et 500 nM.

Les résultats des essais effectués sur les composés de l'invention montrent que *in vitro,* certainds d'entre eux déplacent sélectivement le [³H]flumazénil de ses sites de liaison membranaire vis-à-vis d'une population de récepteurs ω (benzodiazépiniques) associée aux récepteurs GABA_{A} contenant la sous-unité α₅, comparativement aux sous-types de récepteurs ω₁ associés aux récepteurs GABA_{A} contenant la sous-unité α₁, et comparativement à une population de récepteurs ω₂ (benzodiazépiniques de type II) associée aux récepteurs GABA_{A} contenant majoritairement les sous-unités α₂ et α₃.

D'autres composés ont une forte affinité pour les sous-unités α₅, α₁, α₂ et α₃ et ne sont pas sélectifs. En d'autres termes, les composés ont une affinité
. forte pour les sites de liaison membranaire du [³H]flumazénil vis-à-vis d'une population de récepteurs ω (benzodiazépiniques) associée aux récepteurs GABA_{A} contenant la sous-unité α₅,
. forte, moyenne ou faible pour les sous-types de récepteurs ω₁ (benzodiazépiniques de type I) associés aux récepteurs GABA_{A} contenant la sous-unité α₁,
. forte, moyenne ou faible pour une population de récepteurs ω₂ (benzodiazépiniques de type II) associée aux récepteurs GABA_{A} contenant majoritairement les sous-unités α₂ et α₃.

La sélectivité représentée par le rapport CI₅₀ ω₁-cervelet / CI₅₀ ω-hippocampe est comprise entre 1 et 25 et celle représentée par le rapport CI₅₀ ω₂-moëlle / CI₅₀ ω-hippocampe est également comprise entre 1 et 25.

Les composés de l'invention peuvent être utilisés dans le traitement des affections liés aux désordres de la transmission GABAergique des récepteurs GABA_{A} associés à la sous-unité α₅. La distribution préférentielle des récepteurs ω, associés à la sous-unité α₅ du complexe récepteur GABA_{A}, dans le bulbe olfactif, dans des structures limbiques comme l'hippocampe et l'hypothalamus, et dans la moelle épinière, suggère que les composés de l'invention peuvent être utilisés dans le traitement des troubles de l'olfaction, des troubles cognitifs, des troubles hormonaux liés au dysfonctionnement de l'hypothalamus, certains troubles émotionnels et de perception de la douleur. Ils peuvent également être utilisés dans le traitement de la spasticité et des contractures musculaires.

Les composés de l'invention peuvent également être utilisés pour le traitement des affections liées aux désordres de la transmission GABAergique des récepteurs GABA_{A} associés aux sous-unités α₁, α₂ et α₃, c'est-à-dire pour le traitement de l'anxiété, des troubles du sommeil, de l'épilepsie, des troubles du sevrage vis-à-vis de l'alcoolisme. Enfin, ils peuvent être utilisés comme anesthésiques, comme myorelaxants ou comme analgésiques.

A cet effet ils peuvent être présentés sous toutes formes galéniques, associés à des excipients appropriés, pour l'administration entérale ou parentérale, par exemple sous forme de comprimés, dragées, gélules, capsules, solutions ou suspensions buvables ou injectables, suppositoires, etc, dosés pour permettre une administration journalière de 1 à 1000 mg de substance active.

## Revendications

1. Composé répondant à la formule générale (I) 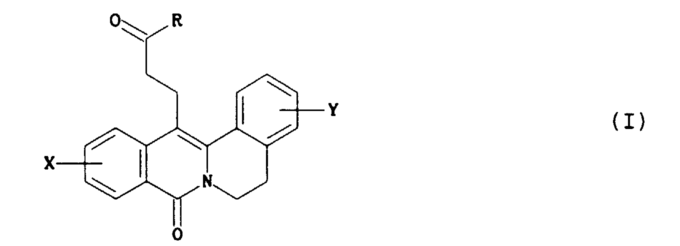 dans laquelle
X représente un atome d'hydrogène ou d'halogène ou un groupe alkyle en C₁-C₃, un ou deux groupes alcoxy en C₁-C₃ ou un groupe trifluorométhyle,
Y représente un atome d'hydrogène ou d'halogène, un groupe alkyle en C₁-C₃ ou un groupe alcoxy en C₁-C₃,
R représente un groupe hydroxy, un groupe méthoxy ou un groupe de formule générale NR₂R₃ dans laquelle R₂ et R₃, indépendamment l'un de l'autre, représentent chacun un atome d'hydrogène, un groupe alkyle en C₁-C₄, un groupe 2-méthoxyéthyle, un groupe 2-aminoéthyle, un groupe 3-aminopropyle, un groupe 2-(diméthylamino)éthyle, un groupe 3-(diméthylamino)propyle ou un groupe 2-pipéridin-1-yléthyle, ou bien encore R₂ et R₃ forment ensemble, et avec l'atome d'azote qui les porte, un cycle morpholinyle, pyrrolidinyle ou pipérazinyle éventuellement substitué en position 4 par un groupe méthyle ou (1,1-diméthyléthoxy)carbonyle,
à l'état de base libre ou de sel d'addition à un acide.

2. Procédé de préparation de composés selon la revendication 1, caractérisé en ce que
· ou bien
on fait réagir un anhydride de formule générale (II) 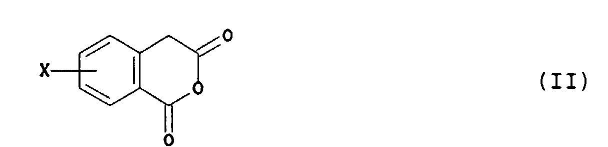 dans laquelle X est tel que défini dans la revendication 1, avec une imine de formule générale (III) 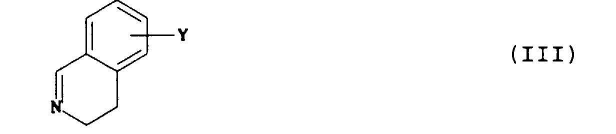 dans laquelle Y est tel que défini dans la revendication 1, pour obtenir un acide de formule générale (IV) 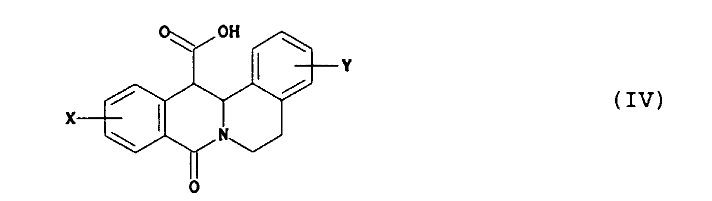 que l'on estérifie par le chlorure de thionyle dans le méthanol, puis on transforme l'ester ainsi obtenu, de formule générale (V) 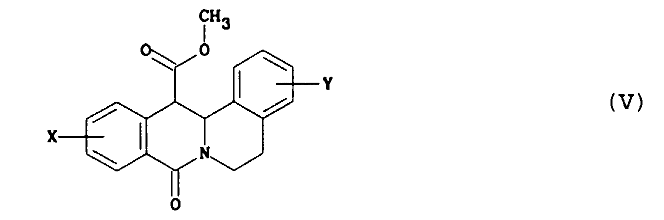 en alcool de formule générale (VI) 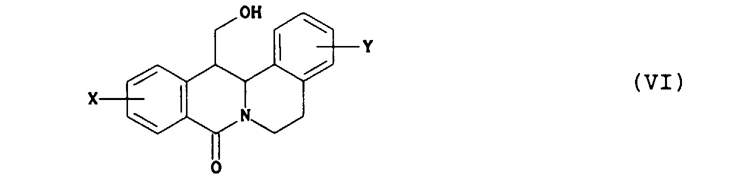 puis on oxyde cet alcool en aldéhyde de formule générale (VII) 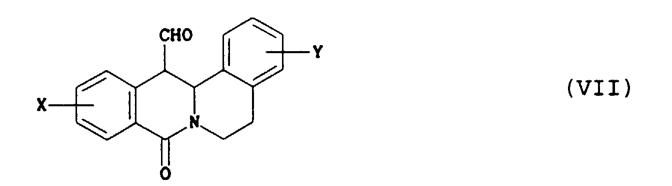 puis on traite cet aldéhyde par le (diméthoxyphosphinyl)acétate de méthyle et on isole l'ester de formule générale (VIII) ainsi obtenu, 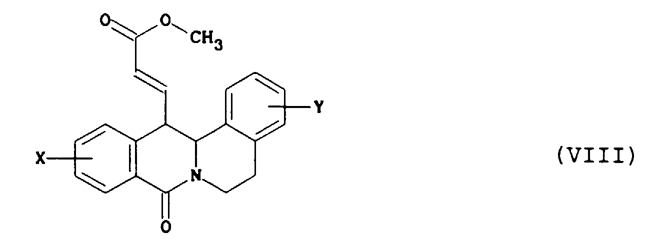 puis on oxyde cet ester en ester de formule générale (IX) 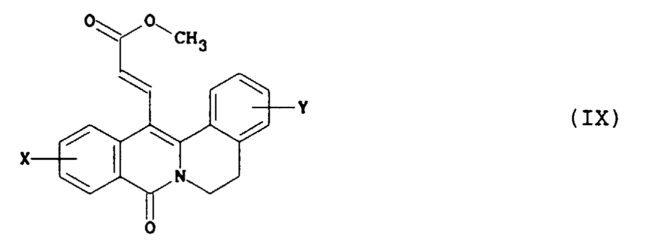 et finalement on soumet cet ester à une hydrogénation catalytique pour obtenir l'ester de formule générale (Ia) 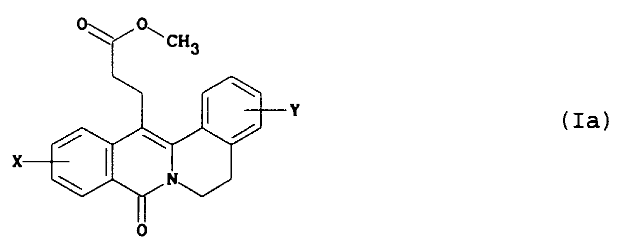 qui correspond à la formule générale (I) où R représente un groupe méthoxy,
puis, si l'on désire un composé de formule générale (I) où R représente un groupe hydroxy, on soumet l'ester de formule générale (Ia) à une hydrolyse pour obtenir un acide de formule générale (Ib) 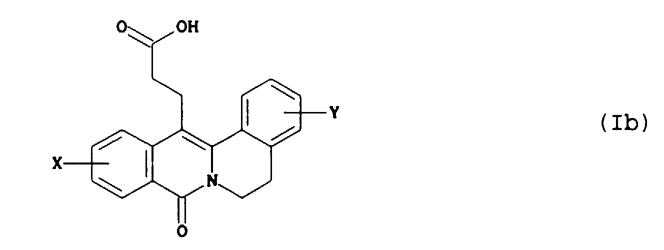 et, si l'on désire un composé de formule générale (I) où R représente un groupe de formule générale NR₂R₃ on traite l'acide de formule générale (Ib) avec une amine de formule générale HNR₂R₃,
· ou bien, dans le cas des composés de formule générale (I) où X et Y sont différents d'un groupe méthoxy, on soumet la dibenzo[*a,g*]quinolizine de formule générale (X) 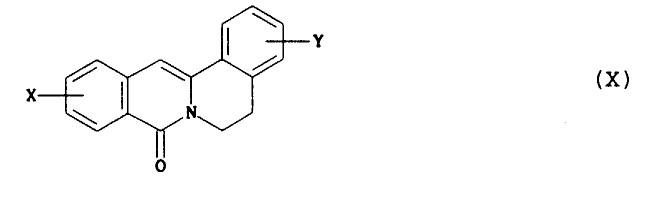 à un traitement par l'oxychlorure de phosphore, pour obtenir l'aldéhyde de formule générale (XI) 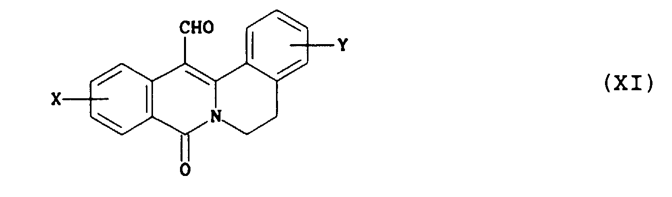 puis on traite ce dernier avec le (diméthoxyphosphinyl)acétate de méthyle pour obtenir l'ester de formule générale (IX), que l'on finit de traiter comme décrit ci-dessus.

3. Médicament caractérisé en ce qu'il consiste en un composé selon la revendication 1.

4. Composition pharmaceutique caractérisée en ce qu'elle contient un composé selon la revendication 1, associé à un excipient.

## Patentansprüche

1. Verbindung der allgemeinen Formel (I) 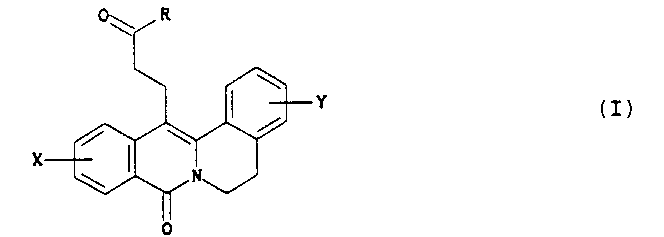 in der
X ein Wasserstoffatom, ein Halogenatom, eine C₁-C₃-Alkylgruppe, eine oder zwei C₁-C₃-Alkoxygruppen oder eine Trifluormethylgruppe,
Y ein Wasserstoffatom, ein Halogenatom, eine C₁-C₃-Alkylgruppe oder eine C₁-C₃-Alkoxygruppe,
R ein Hydroxygruppe, eine Methoxygruppe oder eine Gruppe der allgemeinen Formel NR₂R₃, in der R₂ und R₃ unabhängig voneinanderj eweils ein Wasserstoffatom, eine C₁-C₄-Alkylgruppe, eine 2-Methoxyethylgruppe, eine 2-Aminoethylgruppe, eine 3-Aminopropylgruppe, eine 2-(Dimethylamino)-ethylgruppe, eine 3-(Dimethylamino)-propylgruppe oder eine 2-Piperidin-1-yl-ethylgruppe darstellen oder R₂ und R₃ gemeinsam mit dem sie tragenden Stickstoffatom einen Morpholinyl-, Pyrrolidinyloder Piperazinyl-Ring bilden, der gegebenenfalls in der 4-Stellung durch eine Methylgruppe oder eine (1,1-Dimethylethoxy)-carbonylgruppe substituiert ist, bedeuten,
in Form der freien Base oder eines Säureadditionssalzes.

2. Verfahren zur Herstellung der Verbindungen nach Anspruch 1, dadurch gekennzeichnet, daß man
. entweder ein Anhydrid der allgemeinen Formel (II) 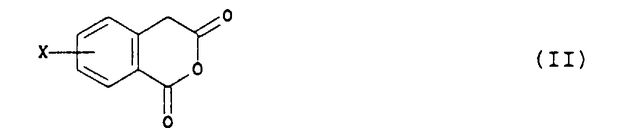 in der X die in Anspruch 1 angegebenen Bedeutungen besitzt,
mit einem Imin der allgemeinen Formel (III) 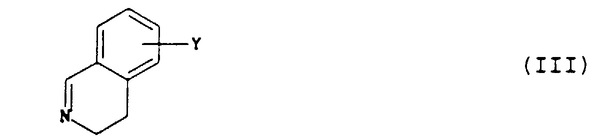 in der Y die in Anspruch 1 angegebenen Bedeutungen besitzt, umsetzt zur Bildung einer Säure der allgemeinen Formel (IV) 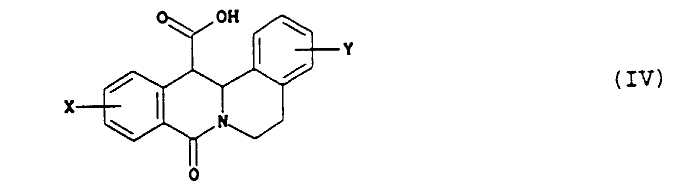 welche man mit Thionylchlorid in Methanol verestert und dann den in dieser Weise erhaltenen Ester der allgemeinen Formel (V) 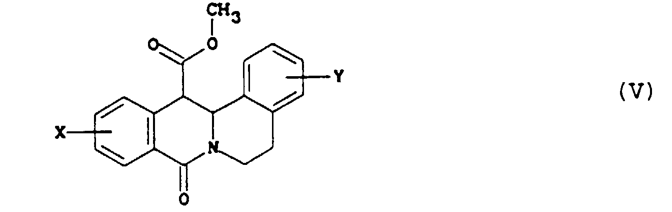 in den Alkohol der allgemeinen Formel (VI) umwandelt 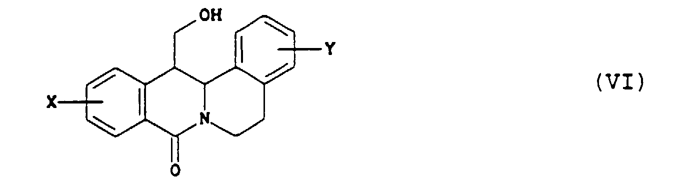 wonach man diesen Alkohol zu einem Aldehyd der allgemeinen Formel (VII) oxidiert 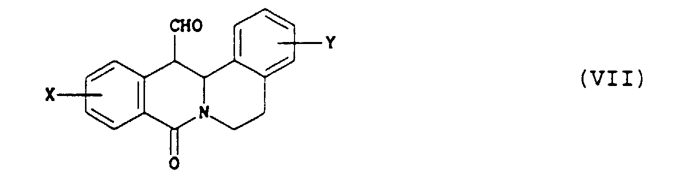 und dann diesen Aldehyd mit (Dimethoxyphosphinyl)-essigsäuremethylester behandelt und den in dieser Weise erhaltenen Ester der allgemeinen Formel (VIII) isoliert 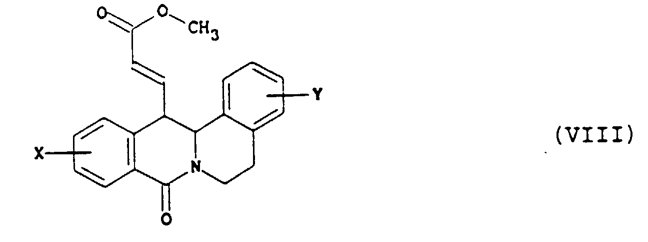 und diesen Ester dann zu dem Ester der allgemeinen Formel (IX) oxidiert 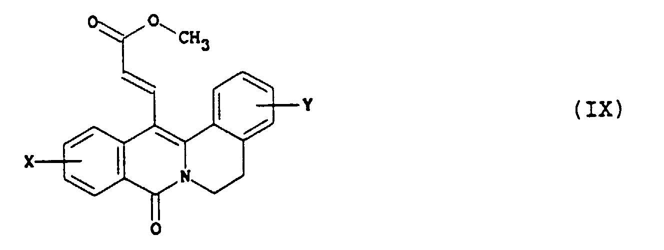 und schließlich diesen Ester einer katalytischen Hydrierung unterwirft zur Bildung des Esters der allgemeinen Formel (Ia) 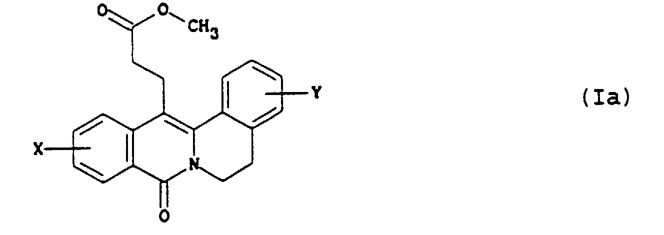 welcher der allgemeinen Formel (I) entspricht, in der R eine Methoxygruppe darstellt.
worauf man, wenn man eine Verbindung der allgemeinen Formel (I) herzustellen wünscht, in der Reine Hydroxygruppe darstellt, man den Ester der allgemeinen Formel (Ia) einer Hydrolyse unterwirft zur Bildung einer Säure der allgemeinen Formel (Ib) 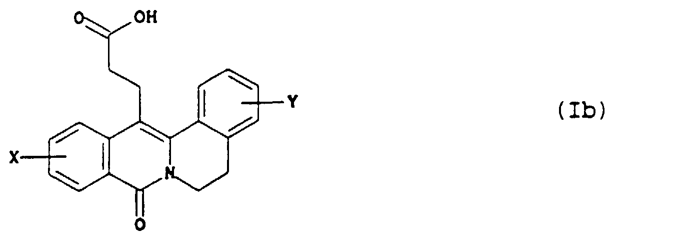 und wenn man eine Verbindung der allgemeinen Formel (I) herzustellen wünscht, in der Reine Gruppe der allgemeinen Formel NR₂R₃ darstellt, man die Säure der allgemeinen Formel (Ib) mit einem Amin der allgemeinen Formel HNR₂R₃ behandelt,
. oder, für den Fall, daß bei den Verbindungen der allgemeinen Formel (I) X und Yvon einer Methoxygruppe verschieden sind, man Dibenzo[a,g]chinolizin der allgemeinen Formel (X) 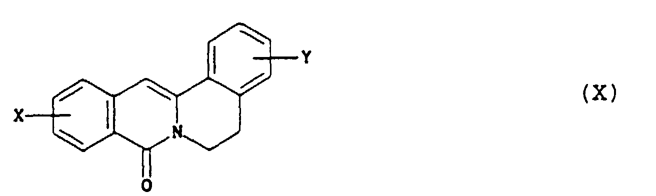 einer Behandlung mit Phosphoroxidchlorid unterwirft zur Bildung des Aldehyds der allgemeinen Formel (XI) 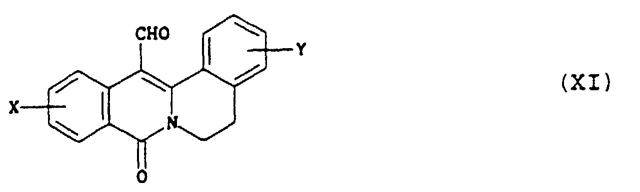 wonach man diesen letzteren mit (Dimethoxyphosphinyl)-essigsäuremethylester behandelt zur Bildung des Esters der allgemeinen Formel (IX), welche man in der oben beschriebenen Weise weiterbehandelt.

3. Arzneimittel, dadurch gekennzeichnet, daß es aus einer Verbindung nach Anspruch 1 besteht.

4. Pharmazeutische Zubereitung, dadurch gekennzeichnet, daß sie eine Verbindung nach Anspruch 1 in Kombination mit einem Trägermaterial enthält.

## Claims

1. Compound corresponding to the general formula (I) 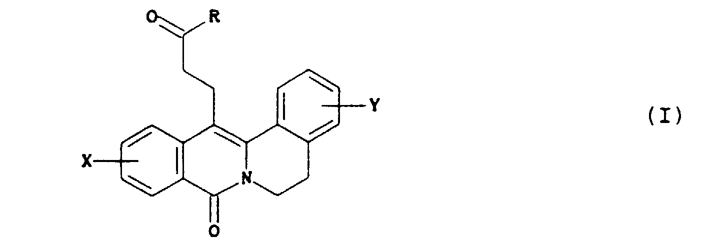 in which
X represents a hydrogen or halogen atom, a C₁-C₃ alkyl group, or one or two C₁-C₃ alkoxy groups or a trifluoromethyl group,
Y represents a hydrogen or halogen atom, a C₁-C₃ alkyl group or a C₁-C₃ alkoxy group,
R represents a hydroxyl group, a methoxy group or a group of general formula NR₂R₃ in which R₂ and R₃, independently of each other, each represent a hydrogen atom, a C₁-C₄ alkyl group, a 2-methoxyethyl group, a 2-aminoethyl group, a 3-aminopropyl group, a 2-(dimethylamino)ethyl group, a 3-(dimethylamino)propyl group or a 2-piperid-1-ylethyl group, or alternatively R₂ and R₃ form, together with the nitrogen atom which bears them, a morpholinyl or pyrrolidinyl ring or a piperazinyl ring optionally substituted in the 4-position with a methyl or (1,1-dimethyl-ethoxy)carbonyl group,
in the form of a free base or an addition salt with an acid.

2. Process for the preparation of compounds according to Claim 1, characterized in that
· either an anhydride of general formula (II) 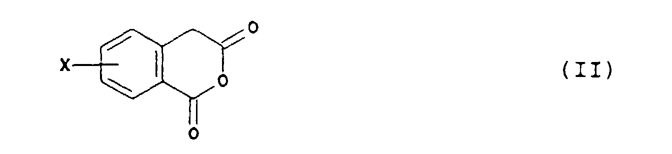 in which X is as defined in Claim 1, is reacted with an imine of general formula (III) 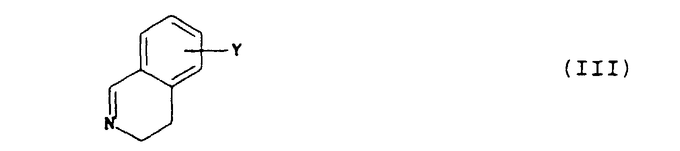 in which Y is as defined in Claim 1, in order to obtain an acid of general formula (IV) 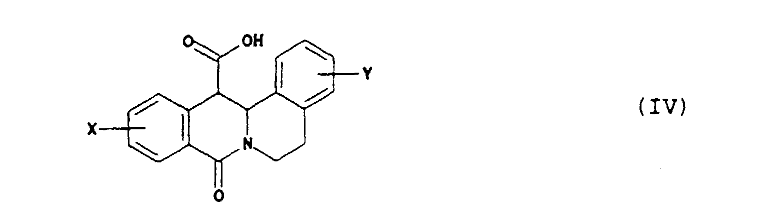 which is esterified with thionyl chloride in methanol, followed by conversion of the ester thus obtained, of general formula (V) 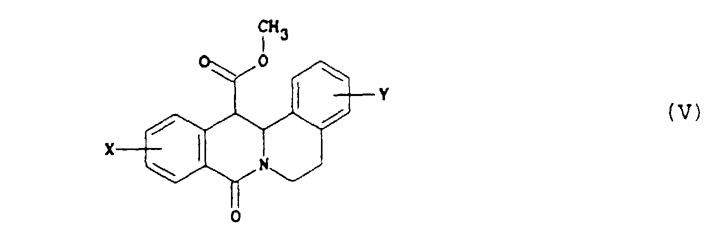 into an alcohol of general formula (VI) 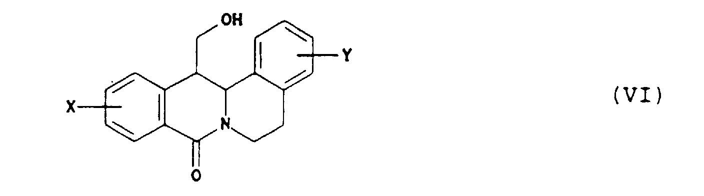 and this alcohol is then oxidized to an aldehyde of general formula (VII) 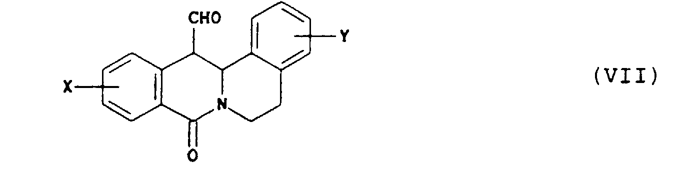 followed by treatment of this aldehyde with methyl (dimethoxyphosphinyl)acetate, and the ester of general formula (VIII) 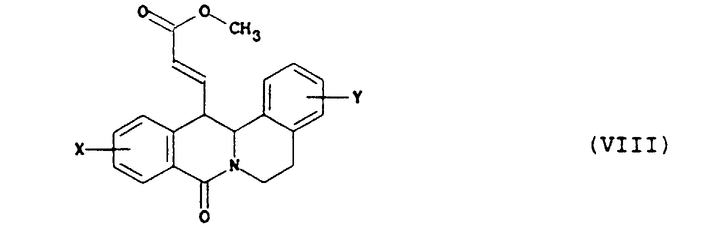 thus obtained is isolated, followed by oxidation of this ester to an ester of general formula (IX) 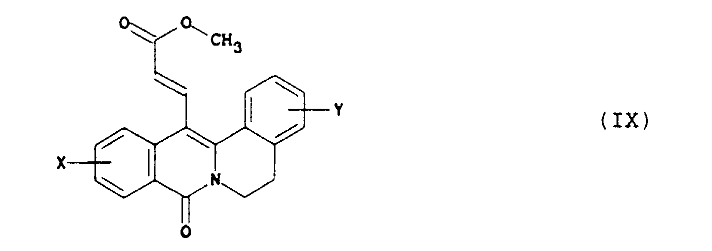 and, lastly, this ester is subjected to catalytic hydrogenation in order to obtain the ester of general formula (Ia) 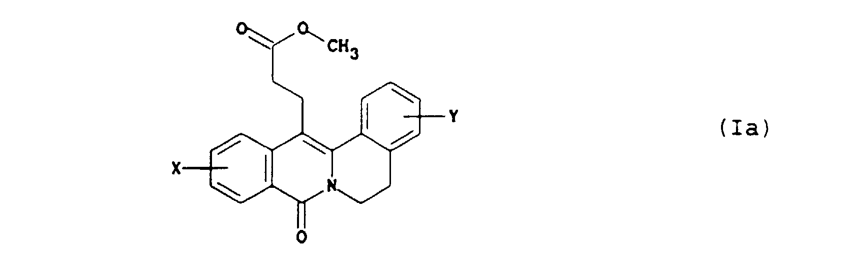 which corresponds to the general formula (I) where R represents a methoxy group,
followed, if it is desired to obtain a compound of general formula (I) where R represents a hydroxyl group, by subjecting the ester of general formula (Ia) to a hydrolysis in order to obtain an acid of general formula (Ib) 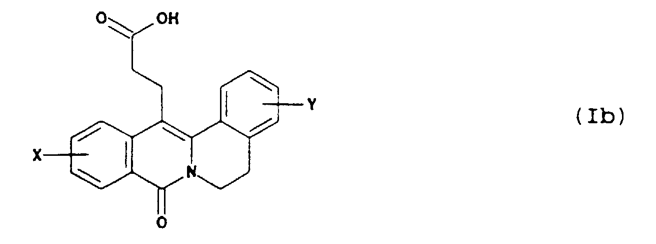 and, if it is desired to obtain a compound of general formula (I) where R represents a group of general formula NR₂R₃, the acid of general formula (Ib) is treated with an amine of general formula HNR₂R₃,
· or, in the case of compounds of general formula (I) where X and Y are other than a methoxy group, the dibenzo[a,g]quinolizine of general formula (X) 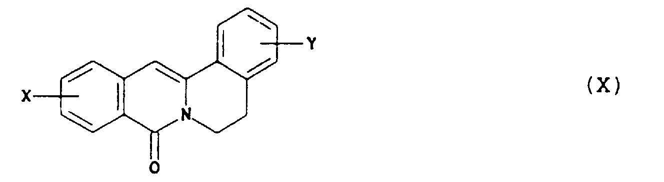 is subjected to a treatment with phosphorus oxychloride, in order to obtain the aldehyde of general formula (XI) 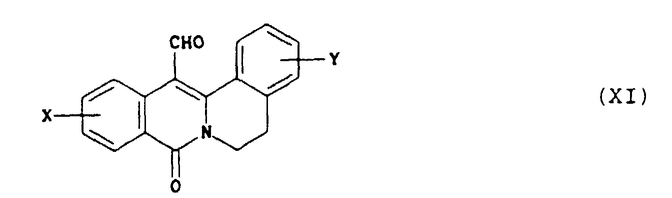 followed by treatment of the latter with methyl (dimethoxyphosphinyl)acetate in order to obtain the ester of general formula (IX), the treatment of which compound is then completed as described above.

3. Medicinal product, characterized in that it consists of a compound according to Claim 1.

4. Pharmaceutical composition, characterized in that it contains a compound according to Claim 1, combined with an excipient.
